# EUROPEAN PATENT APPLICATION

(11) **EP 4 851 272 A1**
(43) Date of publication of application: **09.12.2026**
(21) Application number: 25180824.2
(22) Date of filing: 04.06.2025
(51) Int. Cl.: C12M 1/30, C12M 1/36

(54) **PICKING SYSTEM FOR COLLECTING A SAMPLE FROM A SAMPLE STRUCTURE IN A PICKING PROCESS**

(71) Applicant: Bruker s.r.o., 61900 Brno (CZ)
(72) Inventor: Sliz , Jirí, 664 06 Vinicné Sumice (CZ); Vávra, Tomás, 25601 Benesov (CZ); Karásek, Jan, 66501 Rosice (CZ)
(74) Representative: Gottschald Patentanwälte Partnerschaft mbB

(57) **Abstract**

The invention relates to a picking system for collecting a sample (2) from a sample structure (3) in a picking process, wherein the picking system (1) is configured to receive at least one container (8) with a sample structure (3) thereon and wherein the picking control (13) comprises a picking tool electrode (15) at the picking tool (10), a container electrode (16) at the received container (8) and a voltage supply (17) configured to apply a pulsed voltage (18) between the electrodes (15, 16) to create a current (19) through a current path (20) at least through a non-conducting wall section (21) of the received container (8). It is proposed that the picking control (13) is configured to position the picking tool (10) into the picking motion based on a measured current (23) through the current path (20).

## Description

The present invention relates to a picking system for collecting a sample from a sample structure in a picking process according to claim 1 and to a picking process with such a picking system according to claim 18.

The picking process of collecting a sample from a sample structure and placing the sample onto a target position on a target plate for further cultivation or analysis is a fundamental technique in microbiology. The sample structure normally comprises a culture media and a cell material such as bacterial colonies or fungal colonies. Cell material that is to be collected by the picking system is presently called "target material". The culture media is often provided as an agar plate in the form of a Petri dish. Colony picking is commonly used to isolate single-strain colonies. Historically, colony picking was done manually, with appropriate tools such as a toothpick, needle, pipet tip, or an inoculating loop, to pick up cell material.

The decisive factor for the picking process is that enough cell material is collected for the intended use, without touching other colonies, injuring the surface of the culture media or even picking up culture media (in case of semi-solid or solid culture medium). This requires well-trained personnel, but still, manual processing is prone to human errors and increases the risk of contamination.

Additionally, with the increasing importance of the picking process, for example in clinical laboratories, the manual approach quickly becomes a limiting factor making it inefficient for high-throughput applications. Automation was the next logical development, bringing with it known advantages like reduction of operational costs and the risk of contamination. At the same time, it improves work-flow efficiency, precision and reproducibility.

Automated picking systems comprise a picking control, which is programmed to control a manipulator for positioning a picking tool attached thereto. A challenge is to bring the picking tool into contact with the sample structure without unduly exposing the sample structure to mechanical or electric stress and in particular without unwanted picking up of culture media and/or smearing of cell material over culture media. In detail, gentle and at the same time exact contact sensing is crucial for those automated picking systems. Known automated picking systems are based on optical sensing, for example camera systems, or mechanical sensing, for example touch sensors.

The known prior art (JP S 62273429 A) that builds the basis of the invention is related to a picking system based on contact sensing based on measuring a DC current between the picking tool and the sample structure. This approach requires a first electrode at the picking tool and a second electrode, which is in contact with the sample structure, in particular with an above noted culture media. Bringing the second electrode into contact with the sample structure imposes a challenge in view of system complexity and operational reliability.

It is also known from the state of the art to measure the capacity between the two electrodes, in order to find out about a possible contact condition between two electrodes. While this approach does not require both electrodes to be contacting the sample structure, the complexity of the electronic arrangement to retrieve the capacity is undesirably high.

The invention is based on the problem of improving the known picking system such that a further optimization regarding low system complexity and at the same time high operational reliability is achieved.

The above-noted object is solved by the features of claim 1.

The main realization of the present invention is that if a pulsed voltage is applied between a picking tool electrode and a container electrode, an end-to-end ohmic conductivity between those two electrodes is not necessary in order to detect a contact between the picking tool and the sample structure based on a measured current. This is because the impedance along the current path, which leads at least through a non-conducting wall section of the received container and the sample structure, allows for the establishment of a measurable AC current.

With detecting a contact between the picking tool and the sample structure based on this AC current as a measuring current, it is possible to have a picking tool electrode at the picking tool and a container electrode at the container, which container electrode is generally out of contact from the sample structure. For example, the container electrode may be arranged at a lower surface of the container, while the sample structure is arranged on the upper surface of the container. That way the sample structure and the container electrode are not in material direct contact with each other. This allows for low complexity of the picking system, as far as the detection of a contact between the picking tool and the sample structure goes.

According to the invention, the positioning of the picking tool during the picking process is performed by a picking control based on the measured current noted above. For this, the picking control not only applies the pulsed voltage to the electrodes via a voltage supply, but also measures the current via a current sensor. The measured current provided by the current sensor is a value or signal, that represents the magnitude of the AC current through the above noted current path. This value or signal may be or be derived from the calculated effective current for example. It may also be or be derived from any value, that in any way represents the current through the current path. In any case, if the amplitude of the current is rising or falling, the measured current provided by the current sensor may be rising or falling, respectively.

In detail, a picking system for collecting a sample from a sample structure in a picking process is proposed, wherein the picking system is configured to receive at least one container with a sample structure thereon, in particular in a support of the picking system for the container,
wherein the picking system comprises a picking tool and a manipulator for its motorized positioning,
wherein the picking system comprises a picking control configured to position the picking tool into a picking motion for collecting the sample from the sample structure by controlling the manipulator,
wherein the picking control comprises a picking tool electrode at the picking tool, a container electrode at the received container and a voltage supply configured to apply a pulsed voltage between the electrodes to create a current through a current path at least through a non-conducting wall section of the received container and the sample structure depending on the position of the picking tool,
wherein the picking control comprises a current sensor configured to measure the current through the current path resulting from the pulsed voltage,
wherein for performing the picking process, the picking control is configured to apply a pulsed voltage to the electrodes by the voltage supply, to sense the current between the electrodes by the current sensor and to position the picking tool into the picking motion based on the measured current.

According to claim 2, the tool tip of the picking tool is not only used for engaging the sample structure during collection of the sample, but also for providing one of the electrodes of the measuring unit. This double use of the tip renders the picking systems particularly compact.

The measured current is preferably used by the picking control to control the approach motion of the picking tool to the sample structure according to claim 3. This allows for a quick system reaction to the establishment of the contact between the picking tool and the sample structure, preventing an unintended, deep intrusion of the picking tool into the sample structure. If the sample structure would be composed of a culture media with a cell material on top of that, an unintended deep intrusion of the picking tool would impose the risk of collecting not only the cell material, but also some of the culture media.

As a basis for the evaluation by the picking control, whether the measured current corresponds to a contact condition, a contact criterion is defined. Upon fulfillment of the contact criterion, the approach motion is being terminated. This termination may be an immediate stop of the approach motion or a one or multiple phase brake routine.

Depending on the application, the contact criterion may be defined in various ways. As proposed in claim 4, the contact criterion is simply based on the detection of a minimum rise of the measured current by or to a contact threshold, which contact threshold is preferably defined relatively to a non-contact current. This evaluation can be realized with low effort in terms of electronic circuitry and software.

The non-contact current is preferably derived from a current measurement, while the picking tool is being positioned in a predefined position according to claim 5. This appears to be a simple way to acquire the non-contact current, which may also be understood as a "leakage current".

Preferably, between the approach motion and a depart motion, a scooping motion is realized, which serves to scoop the desired sample from the sample structure (claim 6). This scooping motion requires an additional degree of freedom in movement, preferably along a receiving surface of the container, which receives the sample structure. This scooping motion improves the reliability of the picking process.

The scooping motion may comprise a predefined trajectory, which may be provided by a memory unit of the picking control. Accordingly, in a preferred and simple to realize variant, the picking control simply terminates the scooping motion after having completed such trajectory (claim 7).

However, as the lateral extension of the target material may widely vary, the scooping motion may be controlled based on sensor data according to claim 8. Here and preferably, the scooping motion may be controlled based on the measured current as well. This way it may be prevented, that the scooping motion leads the tool tip of the picking tool out the target material and possibly even into an undesired collection of culture media. Upon fulfillment of the split criterion, the picking tool is preferably positioned into a depart motion according to claim 9.

The depart motion after collecting the sample from the sample structure imposes a challenge to the picking system. This is because during the depart motion, in particular shortly after ending the contact between the picking tool and the sample structure, due to the often soft and sticky consistency of the sample, the effect of stringing may occur, during which elastic strings of the sample material may build up between the picking tool and the sample structure. In order to be able to detect this stringing, a stringing criterion is defined in claim 8.

Alternatively, the stringing threshold is defined based on the knowledge, that with stringing, the measured current may drop less than without stringing (claim 12). If stringing is detected, there is a probability, that strings of sample material remain at the tool tip of the picking tool, which i.a. may lead to undesired contamination at the target plate and in particular cross contamination between target positions.

The sterilization may well be realized by generating a sterilization current through at least a part of the picking tool, which may make use of the voltage supply that is also present within picking control or of a separate component in the form of a separate voltage supply or a separate current supply (claim 13).

As the above noted sterilization routine would also eliminate the collected sample, which would make it necessary to repeat the picking process with the resulting drop in efficiency, it is proposed according to claim 14, to confirm the existence of remaining strings of sample material with a second sensor, namely an optical inspection unit, preferably in the form of a camera. Only if the existence of one or more remaining strings is confirmed, the picking control initiates the above noted sterilization routine. This is a noteworthy contribution to process reliability.

With the proposed solution, a considerable constructive freedom is obtained in view of the overall structure of the picking system. For example, as proposed in claim 15, the picking tool may approach the sample structure from above together with the picking tool electrode, while the container electrode is arranged below the container. With the container electrode being integrated in a support for receiving the container, loading of the picking system with the container may be done with no effort regarding possible wiring of the container electrode. The support for receiving the container may be just a surface for the reception of the container or a mechanical interface for a form fit or a force fit connection with the container.

It may be pointed out, that the pulsed voltage provided by the voltage supply is any kind of pulsed voltage. The easiest to realize is a rectangular voltage as noted in claim 16 as an example. However, a sine-wave voltage may be advantageous, particularly if sensible cell material is being collected.

Claim 17 proposes a preferred embodiment of the current sensor, which is easy and robust at the same time, in particular in the option of rectifying and low pass filtering. Other ways or realizing the current sensor are possible.

Another teaching according to claim 18, which is of equal importance, relates to a method for operating a picking system for collecting a sample from a sample structure according to any one of the preceding claims, wherein the picking system receives at least one container with a sample structure thereon and wherein the picking control positions the picking tool into the picking motion based on the measured current. All explanations given with regard to the picking system are fully applicable to independent claim 19 and dependent claim 20.

In the following, embodiments of the invention are explained with respect to the drawing. The drawings show in
- Fig. 1: a proposed picking system in a perspective view,
- Fig. 2: a simplified illustration of the picking system according to Fig. 1 in a side view,
- Fig. 3: a sequence of a picking process for the picking system according to Fig. 2,
- Fig. 4: the resulting measured current during the sequence according to Fig. 3 with a set of thresholds according a first embodiment and
- Fig. 5: the resulting measured current during the sequence according to Fig. 3 with a set of thresholds according a second embodiment.

The proposed solution is directed to a picking system 1 for collecting a sample 2 from a sample structure 3 in a picking process. The collected sample 2 is then deposited on a target position 4 of a target plate 5 for further processing.

The focus for the proposed solution is the picking process rather than the subsequent processes, such that the explanation of those subsequent processes is no priority at that point.

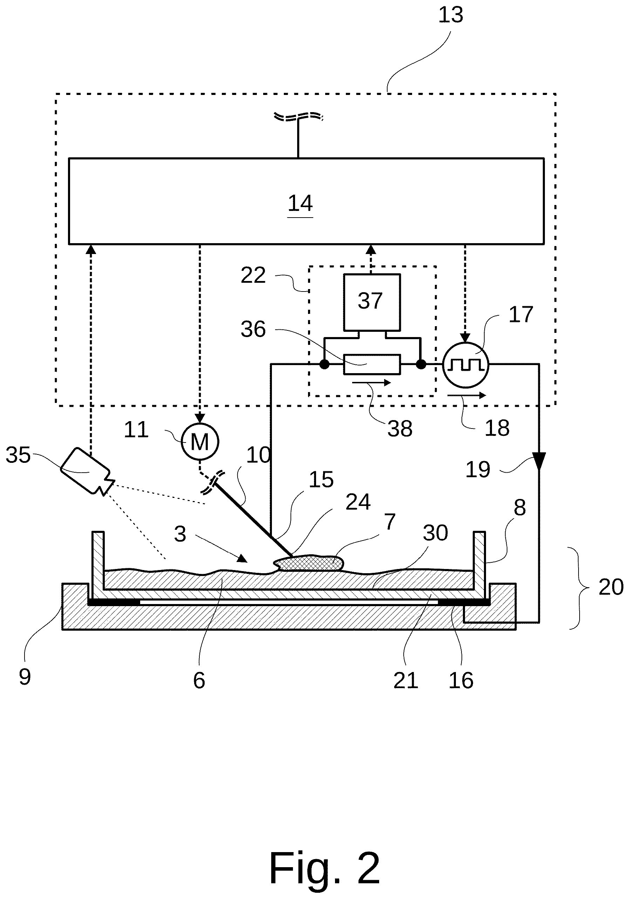

In general, the sample structure 3 comprises a target material, from which target material a sample 2 is to be collected by the picking system 1. Here and preferably, the picking system 1 serves for collecting the sample 2 from a sample structure 3, which sample structure 3 comprises a culture media 6 and a cell material 7, wherein the sample 2 is to be collected from the cell material 7 as target material. The culture media 6 and the cell material 7 is shown in Fig. 2. The culture media 6 may be a liquid culture medium, a solid culture medium or a semi-solid culture medium.

The sample 2 may be a cell material 7 as noted above and may in particular be a microbial cell material, more preferably the microbial cell material contains ,or is mostly comprised out of, microbial cells, even more preferably the microbial cells are whole microbial cells. The microbial cell material may be selected from the group of bacteria cells, archaea cells and fungi cells. In other embodiments the cell material 7 may be selected from the group of mammalian cells. Preferably, the cell material 7 is in the form of a single colony of, in particular microbial, cells.

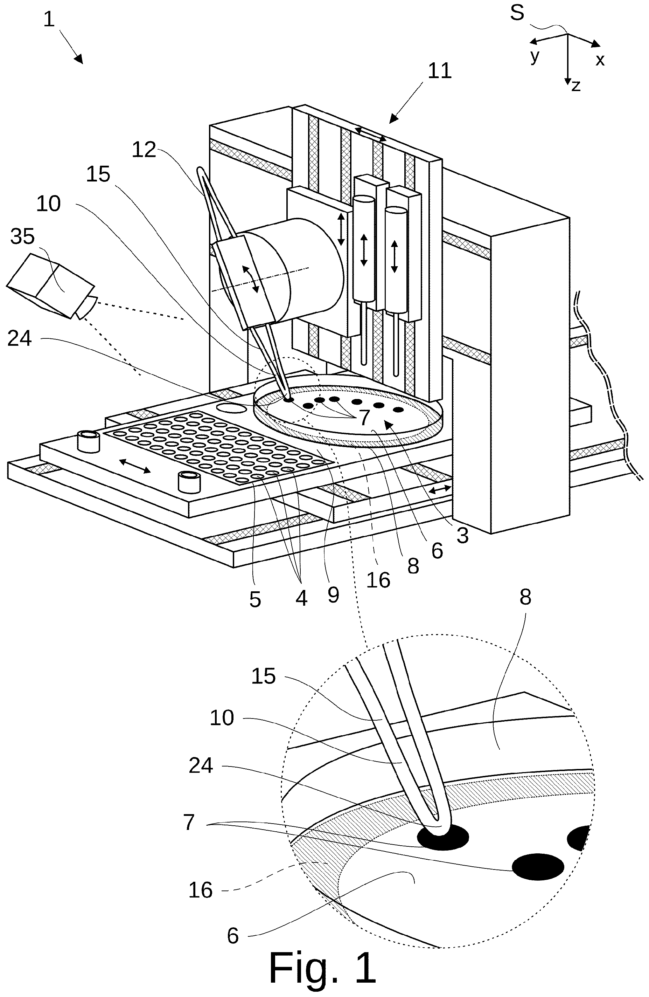

As may be taken from Fig. 1, the picking system 1 is configured to receive at least one container 8 with the sample structure 3 thereon in a support 9. The container 8 here and preferably is realized as a microbial culture plate, more preferably it is realized as a Petri dish. Other types of containers 8 such as flat plates, microtiter plates, vessels, sample tubes etc. may well be applicable.

The picking system 1 comprises a picking tool 10 and a manipulator 11 for its motorized positioning within a coordinate system S defining the x-axis, the y-axis and the z-axis. The manipulator 11 allows positioning of the picking tool 10 linearly in the horizontal (lateral) x-axis and y-axis and in the vertical z-axis as well as rotationally around the y-axis. When the picking tool 10 is approaching the sample structure 3 on the container 8, the picking tool 10 is performing an overall movement from an upper area to a lower area, namely a downward movement, with respect to the vertical direction, here with respect to the z-axis.

The preferred embodiment shown in Fig. 1 comprises not only the picking tool 10, which in Fig. 1 is in contact with the sample structure 3, but also an additional picking tool 12. With the above noted, rotational degree of freedom of the manipulator 11, any one of the picking tools 10, 12 may perform the picking process.

The picking process is being controlled by a picking control 13, which here and preferably is composed of a number of components, which may or may not be concentrated at one location. The picking control 13 is configured to position the picking tool 10 into a picking motion for collecting the sample 2 from the sample structure 3 by controlling the manipulator 11.

Fig. 2 also shows a digital control unit 14, which is at least partly provided by the picking control 13. The digital control unit 14 preferably controls the sequence of actions taken in order to realize the picking process and in particular the picking motion. The digital control unit 14 may be a centralized component as shown in Fig. 2. It may also be realized in a decentralized way by being integrated in various decentralized components.

The picking control 13 comprises a picking tool electrode 15 at the picking tool 10, a container electrode 16 at the received container 8 and a voltage supply 17 configured to apply a pulsed voltage 18 between the electrodes 15, 16. The (peak) value of the pulsed voltage 18 is preferably between 0V and 5V, in particular between 0V and 3.3V. The frequency of the pulsed voltage 18 is preferably between 200kHz and 400kHz, in particular 300kHz.

The electrodes 15, 16 serve to create a current 19 through a current path 20 at least through a non-conducting wall section 21 of the received container 8 and the sample structure 3 depending on the position of the picking tool 10. It is to be understood that the expression "through" is to be understood in the context of an AC current as explained above. The current 19 through the current path 20 is the result of the pulsed voltage 18 being applied to the electrodes 15, 16 and thereby to the impedance established by the current path 20.

The container 8 may be any container 8 suitable to receive a sample structure 3. Preferably, the container 8 is at least partly, in particular completely, made of a non-conducting material such as a plastic material. Usually, the container 8 is provided in the form of a Petri dish. With the arrangement of the container electrode 16 shown in Fig. 2, the non-conducting wall section 21 is the bottom wall rather than the side wall of the container 8, in particular the Petri dish.

The picking control 13 comprises a current sensor 22 configured to measure the current 19 through the current path 20 and between the electrodes 15, 16, respectively, resulting from the pulsed voltage 18. Accordingly, the current sensor 22 is connected to the electrodes 15, 16 by electric wires. Details regarding the current sensor 22 are being provided later.

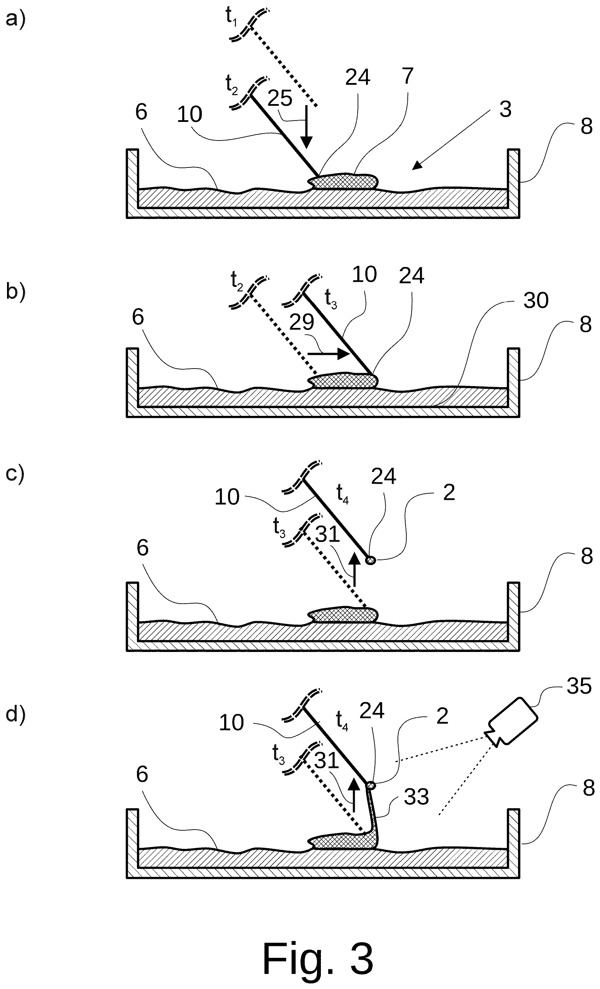

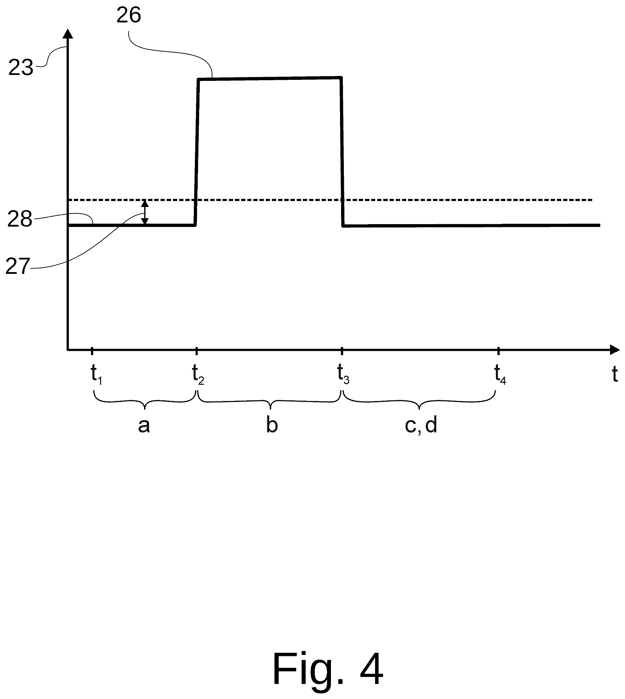

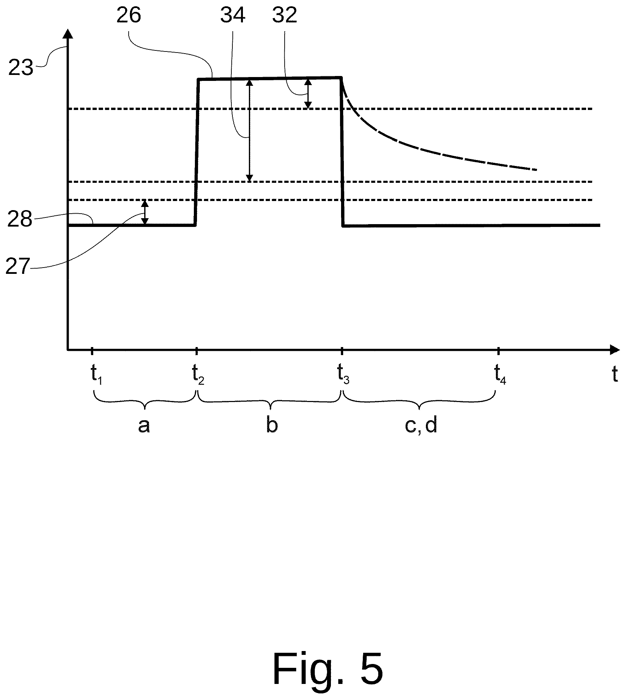

It is essential for performing the picking process, that the picking control 13 is configured to apply a pulsed voltage 18 to the electrodes 15, 16 by the voltage supply 17, to measure the current 19 between the electrodes 15, 16 by the current sensor 22 and to position the picking tool 10 into the picking motion based on the measured current 23. The picking motion during the picking process may be derived from the sequence of Fig. 3 a-d, while the measured current 23 resulting from the pulsed voltage 18 is illustrated in Fig. 4 in a first embodiment and in Fig. 5 in another embodiment.

As noted above, the measured current 23 is a value or signal, that represents the magnitude of the AC current through the above noted current path 20 between the electrodes 15, 16. As Fig. 4 shows, this is a good basis to detect predefined changes in the measured current 23 in order to detect a contact condition between the picking tool 10 and the sample structure 3.

According to one embodiment it is proposed that the picking tool 10 comprises a tool tip 24 shown in the detailed illustration in Fig. 1. The tool tip 24 provides a double function: Firstly, it serves for engaging the sample structure 3 during collecting the sample 2 from the sample structure 3 and secondly it provides the picking tool electrode 15. Here and preferably, the tool tip 24 of the picking tool 10 is provided by a looped wire which allows for sterilization by having a current flow through the looped wire with the resulting generation of heat.

The first phase of the picking motion preferably is an approach motion 25, which begins in a starting position of the picking tool 10 and which ends in a contact position, in which the contact condition between the picking tool 10 and the sample structure 3 is established. The approach direction of the approach motion 25 may be vertical as shown in Fig. 3a, or inclined with respect to the vertical direction, here the direction of the z-axis.

With the picking tool 10 preferably being of longitudinal shape along a longitudinal axis, the picking tool 10 may be inclined with respect to the horizontal direction during the picking motion, in particular during the approach motion 25. The inclination may be advantageous as with the inclination, the contact surface between the tool tip 24 and the sample structure 3 may be enlarged, which enlarged contact surface reduces the contact resistance for the picking tool electrode 15. The inclination angle with respect to the vertical direction is preferably between 10 degrees and 60 degrees.

In an alternative, the approach motion 25 at least shortly before establishing the contact condition may have an approach direction along the longitudinal axis of the picking tool 10, which longitudinal axis may be inclined as noted above.

As noted above, during the picking motion, the picking tool 10 and with it the picking tool electrode 15 is approaching the sample structure 3 in the approach motion 25. During the approach motion 25 of the picking tool 10, the picking control 13 monitors the measured current 23 in view of a contact criterion. The contact criterion is defined such that fulfillment of the contact criterion indicates a contact condition between the picking tool 10 and the sample structure 3 such that an appropriate reaction may be initiated. Here and preferably, upon fulfillment of the contact criterion, the picking control 13 terminates the approach motion 25 of the picking tool 10.

There are various concepts possible for defining the contact criterion. After having established a contact between the tool tip 24 and the sample structure 3, the measured current 23 through the current path 20 will rise to the measured contact current 26 shown in Fig. 4. Accordingly, in a preferred embodiment, the contact criterion is defined as the measured current 23 through the current path 20 at least rising by or to a contact threshold 27. The contact threshold 27 may be defined as an absolute threshold or as a relative threshold. Here and preferably, the contact threshold 27 is a relative threshold, namely more than 10%, preferably more than 15% and most preferably 20% relative to a non-contact current 28 through the current path 20.

The non-contact current 28 is the current 19 through the current path 20 in a non-contact condition between the picking tool 10 and the sample structure 3. Preferably, the non-contact current 28 is the current 19 through the current path 20 in a non-contact condition between the picking tool 10 and the sample structure 3, when the picking tool 10 is being positioned in a predefined non-contact position, preferably in the above noted starting position. The predefined non-contact position may preferably be an absolute position within the coordinate system S such as the starting position, or any predefined relative position. The contact threshold 27 and the non-contact current 28 are shown in Fig. 4, for example.

Preferably, with the picking tool 10 being positioned in the predefined position, preferably in the starting position, the picking control 13 is configured to apply a pulsed voltage 18 to the electrodes 15, 16 by the voltage supply 17, to measure the current 19 between the electrodes 15, 16 by the current sensor 22 and to store the measured current 19 as the non-contact current 28. The stored non-contact current 28 may well be used later during the picking process, as will be explained. It is preferred, that the non-contact current 28 is measured and stored for each and any picking motion.

The contact criterion may additionally include a rule regarding the dynamic of the measured current 23. For example, the contact criterion may additionally include the requirement, that the gradient of the measured current 23 with respect to time exceeds a certain dynamics threshold. The inclusion of this dynamic aspect is well applicable for all criteria being discussed in the present disclosure.

After having detected the contact condition and after having terminated the approach motion 25, there are several actions possible. One preferred option, which is shown in Fig. 3b, is that during the picking motion, after termination of the approach motion 25, the picking tool 10 scoops a sample 2 from the sample structure 3, here and preferably the cell material 7, in a scooping motion 29 along a receiving surface 30 of the container 8, which receives the sample structure 3. Preferably, the scooping motion 29 may be a horizontal motion, which in the shown embodiment is a lateral motion with regard to the z-axis.

The above noted scooping motion 29 guarantees effective collection of the desired sample 2. However, depending on the consistency of the sample structure 3, it may be advantageous to simply depart from the sample structure 3 in a depart motion 31 instead of performing the scooping motion 29. While the scooping motion 29 is, preferably, mainly a horizontal motion, the depart motion 31 is, preferably, mainly a vertical motion. As noted above, the scooping motion 29 may comprise a predefined trajectory for any picking process, such that the picking control 13 terminates the scooping motion 29 after having completed the predefined scooping motion trajectory. However, a preferred sensor based scooping motion 29 is presented in the following.

For the determination of the, preferably horizontal, extent of the scooping motion 29 various strategies are possible. In a simple option, the extent of the scooping motion 29 is predefined and so small, that for the intended application, no moving out of the target material, here and preferably the cell material 7, is to be expected. In another option, the extent is determined in dependence of the horizontal extension of the target material. For this determination, the above noted measured current 23 through the current path 20 may well be applied, as will be explained in the following.

According to one embodiment it is proposed that during the scooping motion 29 of the picking tool 10 (Fig. 3b), the picking control 13 monitors the measured current 23 in view of a split criterion, which indicates the picking tool 10 coming out of contact from the sample structure 3, and that upon fulfillment of the split criterion, the picking control 13 terminates the scooping motion 29 of the picking tool 10. This means, that the horizontal extension of the scooping motion 29 may be as large as the horizontal extension of the target material, here and preferably the cell material 7, allows.

The split criterion is preferably defined as the measured current falling under the contact threshold 27, preferably the above noted contact threshold 27 based on the stored non-contact current 28, as shown in Fig. 4. Alternatively, the split criterion may be defined as the measured current 23 at least dropping by or to a split threshold 32 as shown in Fig. 5, which split threshold 32 is higher than the contact threshold 27. Again, the split threshold 32 may be defined as an absolute threshold or a relative threshold. Here and preferably, the split threshold 32 is defined as a drop in the measured current 23 of at least 50% relative to the measured contact current 26. Other definitions of the split criterion are possible.

As shown in Fig. 3c, during the picking motion, after termination of the scooping motion 29, the picking tool 10 departs from the sample structure 3 in a depart motion 31, which here and preferably is a mainly vertical motion. As an alternative, the depart direction of this depart motion 31 may be inclined as noted above.

It may be pointed out, that the approach motion 25, the scooping motion 29 and the depart motion 31 may be realized as separate motions, being separated by a stop of motion in between them. It may, however, be foreseen that the transitions between the approach motion 25, the scooping motion 29 and the depart motion 31 are being smoothed such that those three phases of the picking motion appear as one single steady motion. In this embodiment, the termination of the approach motion 25 and of the scooping motion 29 would include a braking phase before actually stopping the motion. Accordingly, the contact criterion, which fulfillment would cause termination of the approach motion 25, would preferably be defined as a first rise of the current 19 through the current path 20, such that the termination process would be initiated early enough.

As noted above, during the depart motion 31, stringing 33 between the tool tip 24 and the sample material may occur, which may lead to undesirable contamination and cross-contamination scenarios. Therefore, it is preferred that during the depart motion 31, the picking control 13 monitors the measured current 23 in view of a stringing criterion, which indicates stringing 33 of sample material during the depart motion 31, wherein upon fulfillment of the stringing criterion, the picking control 13 performs a sterilization routine and as a result rejects the sample 2 and repeats the picking process.

Referring to Fig. 4, the stringing criterion is defined as the measured current 23 staying above the contact threshold 27, preferably the above noted contact threshold 27 based on the stored non-contact current 28, during at least a first part of the depart motion.

As an alternative shown in Fig. 5, the stringing criterion may also be based on the assumption, that the establishment of strings 33 of sample material, here and preferably cell material 7, between the collected sample 2 and the sample structure 3 remaining on the container 8 leads to a reduced drop of the current 19 through the current path 20 when dissolving the contact condition between the tool tip 24 and the sample structure 3 on the container 8. This reduced drop of the current 19 is indicated in Fig. 5 in dotted line. It is then preferred, that the stringing criterion is defined as the measured current 23 at most dropping by or to a stringing threshold 34, which stringing threshold 34 is above the contact threshold 27. Again, the stringing threshold 34 may be defined as an absolute threshold or a relative threshold.

The monitoring of the measured current 23 in view of the stringing criterion is preferably performed directly with the depart motion 31. However, it may also be advantageous to perform the measured current 23 in view of the stringing criterion at a certain time or position of the picking tool 10 during the depart motion 31.

For the realization of the sterilization routine, a number of preferred options are applicable. However, here and preferably, a thermal sterilization is proposed. According to this, during the sterilization routine, the picking control 13 generates a sterilization current through at least a part of the picking tool 10. Here and preferably, the voltage supply 17 is providing the sterilization current by applying a sterilization voltage to the picking tool 10. As an alternative, a separate component, in particular a current supply, is providing the sterilization current to the picking tool 17.

If the tool tip 24 of the picking tool 10 is provided by a looped wire as noted above, the sterilization current may flow through the looped wire. The sterilization voltage and/or the sterilization current may be pulsed or non-pulsed.

The thermal sterilization based on a sterilization current through the tool tip 24 of the picking tool 10 is an easy to realize and cost-effective solution. Due to the generated heat at the tool tip 24, a collected sample 2 that is subject to above noted stringing 33 may easily be burned off the tool tip 24.

In some situations, noted above, despite an initial stringing 33 having occurred, still no strings 33 may remain at the tool tip 24. For example, the established strings 33 may have disappeared during the depart motion 31 due to elastic effects. In this case, an above noted sterilization routine for burning off the sample 2 is not necessary. Accordingly it is preferred, that the picking system 1 comprises an optical inspection unit 35 for detecting remaining strings 33 of sample material at the picking tool 10, in particular at the tool tip 24, and that upon fulfillment of the stringing criterion, the picking control 13 initiates the sterilization routine only if the inspection routine proves the existence of remaining strings 33 at the picking tool 10. The prove of the existence of remaining strings 33 may be performed based on image recognition. Alternatively, the optical inspection system may well be used to prove the non-existence of remaining strings 33, which prove would lead to following up with the picking process without performing a sterilization routine.

With the above noted two step approach, performing a sterilization routine is performed only if actually needed. This saves costs and time, especially when taking into account, that the optical inspection unit 35 may be part of the picking system 1 anyhow, namely, in order to identify target material within the container 8 as a basis for laterally positioning the picking tool 10 during the approach motion 25.

Taking a look at the structure of the picking system 1 shown in Fig. 1 it becomes clear, that the overall mechanical structure has various advantages.

According to this structure, during the approach motion 25 of the picking tool 10, the picking tool 10 approaches the sample structure 3 from above the container 8, while the container electrode 16 is arranged below the container 8, here and preferably integrated in the support 9 for receiving the container 8. This means, that for loading the picking system 1 with the container 8 including the sample structure 3, the container 8 simply has to be placed onto the container electrode 16. The container electrode 16 may be integrated into a support 9 of the picking system 1 for the container 8, such that the above noted loading does not require any wiring for contacting the container electrode 16 as noted above.

Here and preferably, the container electrode 16 is of the form of a ring, such that optical inspection and illumination of the container 8 from below is possible. It is also possible, to realize the container electrode 16 as a transparent coating of the support 9 for the container 8, such that a ring shape is not necessary.

However, it may be advantageous in some applications to fixedly attach the container electrode 16 to the container 8 itself. For example, in one option it is then preferred that the container electrode 16 is attached to the lower side of the non-conducting wall section 21 of the container 8. In addition, or as an alternative, it is preferred, that the container electrode 16 is metalized on a lower side of the non-conducting wall section 21 of the container 8.

As already noted, the pulsed voltage 18 may be realized in various pulse forms. In the simplest variant it is proposed that the pulsed voltage 18 is a pulsed DC voltage in the form of a rectangular voltage. Alternatively, the pulsed voltage 18 may be a triangular voltage. As another alternative, the pulsed voltage 18 may be a saw tooth voltage. Finally, as another alternative, the pulsed voltage 18 may be a sine-wave voltage. Other pulse forms for the pulsed voltage 18 are possible.

The illustration of Fig. 2 depicts a preferred embodiment for the current sensor 22 of the picking system 1. Here it is proposed that the current sensor 22 comprises a sense resistor 36 in an electric connection between one of the electrodes 15, 16 and the voltage supply 17, wherein the current sensor 22 comprises a measuring circuit 37 to derive the measured current 23 from the measuring voltage 38 over the sense resistor 36. As the current 19 through the current path 20 is an AC current, the measuring voltage 38 which drops across the sense resistor 36, is an AC voltage accordingly. The picking control 13 derives the measured current 23 by rectification of the measuring voltage 38 over the sense resistor 36 and, preferably, subsequent filtering, further preferably low pass filtering, of the rectified voltage. Other concepts of current measuring are well applicable to measuring the current 19 through the current path 20.

It has been explained that the picking system 1 shown in Fig. 1 comprises not only the one picking tool 10, that has been discussed in detail above, but also an additional picking tool 12. All explanations given to the above noted picking tool 10 are fully applicable to the additional picking tool 12. This embodiment with two picking tools 10, 12 has a noteworthy advantage regarding the above noted, thermal sterilization routine based on sterilization current through the tool tip 24. As noted above, with the rotational degree of freedom of the manipulator 11, any one of the picking tools 10, 12 may perform the next picking process. Taking into account, that the above noted sterilization routine requires a cooling off time of the tool tip 24 before being able to be used for the next picking process, it is possible to use the respective other one of the picking tools 10, 12 instead to perform the next picking process.

Another teaching which is of equal importance relates to a method for performing a picking process with a proposed picking system 1.

In detail, it is proposed, that the picking system 1 receives at least one container 8 with a sample structure 3 thereon, that during the picking process, a pulsed voltage 18 is being applied to the electrodes 15, 16 by the voltage supply 17, the current 19 between the electrodes 15, 16 is being measure by the current sensor 22 and the picking tool 10 is being positioned into the picking motion based on the measured current 23. This method utilizes the picking system 1 according to the first teaching, such that reference is made to all explanations given with regard to the picking system 1 and its operation.

## Claims

1. Picking system for collecting a sample (2) from a sample structure (3) in a picking process, wherein the picking system (1) is configured to receive at least one container (8) with a sample structure (3) thereon,
wherein the picking system (1) comprises a picking tool (10) and a manipulator (11) for its motorized positioning,
wherein the picking system (1) comprises a picking control (13) configured to position the picking tool (10) into a picking motion for collecting the sample (2) from the sample structure (3) by controlling the manipulator (11),
wherein the picking control (13) comprises a picking tool electrode (15) at the picking tool (10), a container electrode (16) at the received container (8) and a voltage supply (17) configured to apply a pulsed voltage (18) between the electrodes (15, 16) to create a current (19) through a current path (20) at least through a non-conducting wall section (21) of the received container (8) and the sample structure (3) depending on the position of the picking tool (10),
wherein the picking control (13) comprises a current sensor (22) configured to measure the current (19) through the current path (20) resulting from the pulsed voltage (18),
wherein for performing the picking process, the picking control (13) is configured to apply a pulsed voltage (18) to the electrodes (15, 16) by the voltage supply (17), to measure the current (19) between the electrodes (15, 16) by the current sensor (22) and to position the picking tool (10) into the picking motion based on the measured current (23).

2. Picking system according to claim 1, **characterized in that** the picking tool (10) comprises a tool tip (24) for engaging the sample structure (3) during collecting the sample (2) from the sample structure (3) and that the tool tip (24) provides the picking tool electrode (15), preferably, that the tool tip (24) of the picking tool (10) is provided by a looped wire.

3. Picking system according to claim 1 or 2, **characterized in that** during the picking motion, the picking tool (10) and with it the picking tool electrode (15) is approaching the sample structure (3) in an approach motion (25) and that during the approach motion (25) of the picking tool (10), the picking control (13) monitors the measured current (23) in view of a contact criterion, which indicates a contact condition between the picking tool (10) and the sample structure (3), and that upon fulfillment of the contact criterion, the picking control (13) terminates the approach motion (25) of the picking tool (10).

4. Picking system according to claim 3, **characterized in that** the contact criterion is defined as the measured current (23) at least rising by or to a contact threshold (27), preferably, that the contact threshold (27) is more than 10%, preferably more than 15% and most preferably 20% relative to a non-contact current (28) in a non-contact condition between the picking tool (10) and the sample structure (3).

5. Picking system according to claim 4, **characterized in that** the non-contact current (28) is the current (19) through the current path (20) in a non-contact condition between the picking tool (10) and the sample structure (3), with the picking tool (10) being positioned in a predefined position, preferably in a starting position, preferably, that the picking control (13) is configured to apply a pulsed voltage (18) to the electrodes (15, 16) by the voltage supply (17) with the picking tool (10) being positioned in a predefined position, preferably in a starting position, to measure the current (19) between the electrodes (15, 16) by the current sensor (22) and to store the measured current (19) as the non-contact current (28).

6. Picking system according to one of the claims 3 to 5, **characterized in that** during the picking motion, after termination of the approach motion (25), the picking tool (10) scoops a sample (2) from the sample structure (3) in a scooping motion (29), in particular along a receiving surface (30) of the container (8), or the picking tool (10) departs from the sample structure (3) in a depart motion (31).

7. Picking system according to one of the claims 3 to 6, **characterized in that** the picking control (13) terminates the scooping motion (29) after having completed a predefined scooping motion trajectory.

8. Picking system according to one of the claims 3 to 6, **characterized in that** during the scooping motion (29) of the picking tool (10), the picking control (13) monitors the measured current (23) in view of a split criterion, which indicates the picking tool (10) coming out of contact from the sample structure (3), and that upon fulfillment of the split criterion, the picking control (13) terminates the scooping motion (29) of the picking tool (10), preferably, that the split criterion is defined as the measured current (23) at least dropping by or to a split threshold (32).

9. Picking system according to claim 7 or 8, **characterized in that** during the picking motion, after termination of the scooping motion (29), the picking tool (10) departs from the sample structure (3) in a depart motion (31).

10. Picking system according to claim 6 or 9, **characterized in that** during the depart motion (31), the picking control (13) monitors the measured current (23) in view of a stringing criterion, which indicates stringing (33) of sample material during the depart motion (31), wherein upon fulfillment of the stringing criterion, the picking control (13) performs a sterilization routine.

11. Picking system according to claim 10, **characterized in that** the stringing criterion is defined as the measured current (23) staying above the contact threshold (27) during at least a first part of the depart motion.

12. Picking system according to claim 10, **characterized in that** the stringing criterion is defined as the measured current (23) at most dropping by or to a stringing threshold (34) during at least a first part of the depart motion, which stringing threshold (34) is above the contact threshold.

13. Picking system according to claim 10 and, if so, to claim 11 or 12, **characterized in that** during the sterilization routine, the picking control (13) generates a sterilization current through at least a part of the picking tool (10), preferably, that the picking control (13) generates the sterilization current by applying a sterilization voltage to the picking tool (10).

14. Picking system according to claim 10 and, if so, to one of the claims 11 to 13, **characterized in that** the picking system (1) comprises an optical inspection unit (35) for detecting remaining strings (33) of sample material at the picking tool (10) and that upon fulfillment of the stringing criterion, the picking control (13) initiates the sterilization routine only if the inspection routine proves the existence of remaining strings (33) at the picking tool (10).

15. Picking system according to one of the preceding claims, **characterized in that** during the approach motion (25) of the picking tool (10), the picking tool (10) approaches the sample structure (3) from above the container (8), while the container electrode (16) is arranged below the container (8), preferably, that the picking system (1) comprises a support (9) for receiving the container (8) and that the container electrode (16) is integrated in the support (9).

16. Picking system according to one of the preceding claims, **characterized in that** the pulsed voltage (18) is a pulsed DC voltage in the form of a rectangular voltage, or, that the pulsed voltage (18) is a triangular voltage, or, that the pulsed voltage (18) is a saw tooth voltage, or, that the pulsed voltage (18) is a sine-wave voltage.

17. Picking system according to one of the preceding claims, **characterized in that** the current sensor (22) comprises a sense resistor (36) in an electric connection between one of the electrodes (15, 16) and the voltage supply (17) and that the current sensor (22) comprises a measuring circuit (37) to derive the measured current (23) from the measuring voltage (38) over the sense resistor (36), preferably, that the picking control (13) derives the measured current (23) by rectification of the measuring voltage (38) over the sense resistor (36) and, preferably, subsequent filtering, further preferably low pass filtering, of the rectified voltage.

18. Method for performing a picking process with a picking system (1) according to any one of the preceding claims, wherein the picking system (1) receives at least one container (8) with a sample structure (3) thereon,
wherein during the picking process, a pulsed voltage (18) is being applied to the electrodes (15, 16) by the voltage supply (17), the current (19) between the electrodes (15, 16) is being measured by the current sensor (22) and the picking tool (10) is being positioned into the picking motion based on the measured current (23).

19. Method according to claim 18, **characterized in that** during the picking motion, the picking tool (10) is approaching the sample structure (3) in an approach motion (25) and that during the approach motion (25) of the picking tool (10), the picking control (13) monitors the measured current (23) in view of a contact criterion, which indicates a contact condition between the picking tool (10) and the sample structure (3), and that upon fulfillment of the contact criterion, the picking control (13) terminates the approach motion (25) of the picking tool (10).
